# EUROPEAN PATENT APPLICATION

(11) **EP 3 650 484 A1**
(43) Date of publication of application: **13.05.2020**
(21) Application number: 18827433.6
(22) Date of filing: 06.07.2018
(51) Int. Cl.: C08G 69/48, A61K 31/198, A61K 31/401, A61K 45/00, A61K 47/59, A61K 47/60, A61P 13/12, C08B 37/02, C08B 37/08

(54) **KIDNEY-TARGETING DRUG DELIVERY CARRIER**

(30) Priority: 06.07.2017 JP 2017132806
(71) Applicant: Kyoto Pharmaceutical University, Kyoto-shi, Kyoto 607-8414 (JP)
(72) Inventor: KATSUMI, Hidemasa, Kyoto-shi Kyoto 607-8414 (JP); YAMAMOTO, Akira, Kyoto-shi Kyoto 607-8414 (JP)
(74) Representative: Weickmann & Weickmann PartmbB
(86) International application number: PCT/JP2018/026414
(87) International publication number: WO 2019/009436

(57) **Abstract**

The present invention relates to a compound having a macromolecular carrier having a plurality of terminal groups, wherein the carbonyl group of serine is linked by a peptide bond or an ester bond directly or via a linker to the terminal groups, a carrier for drug delivery composed of the compound, and a medicament for preventing or treating renal diseases, containing the carrier for drug delivery and a drug bonded to the carrier directly or via a linker or encapsulated therein. According to the present invention, a carrier for drug delivery that is selectively accumulated in kidney in vivo can be provided.

## Description

### [Technical Field]

The present invention relates to a carrier for kidney targeting drug delivery that selectively accumulates in kidney, particularly, proximal renal tubule, in the body, and a kidney targeting medicament containing a drug bonded to or encapsulated in the carrier.

### [Background Art]

The development of a dosage form for most effectively and safely administering a drug by controlling the pharmacokinetics of the drug, that is, a drug delivery system, has attracted attention in recent years in drug development. However, carriers for drug delivery that selectively accumulate in the kidney have hardly been developed.

For example, drugs modified with succinic acid or aconitic acid are known to accumulate relatively easily in the kidney, but they also accumulate in the liver at the same time (non-patent document 1).

In addition, polyvinylpyrrolidone-type compounds have drawn attention as targeting elements that selectively accumulate in the kidney. However, since they are artificial polymers, decomposition thereof is difficult after being distributed to the kidney, thus raising a concern about accumulation properties and safety. Therefore, clinical application thereof as kidney targeting elements is difficult and has not been put to practical use (non-patent document 2).

Renal disease includes various diseases such as glomerulonephritis, IgA nephropathy, diabetic nephropathy, membranous nephropathy, hydronephrosis, contrast nephropathy, pyelonephritis, renal failure, acute nephritis, chronic nephritis, uremia, interstitial nephritis, kidney disease, nephrotic syndrome, hypertensive nephrosclerosis, diabetic glomerulosclerosis, kidney calculus, amyloid kidney, kidney intravenous thrombosis, Alport syndrome, kidney tumor and the like. Therefore, development of a carrier for drug delivery that selectively accumulates in the kidney is extremely important in treating these diseases.

### [Document List]

### [non-patent documents]

non-patent document 1: Yamasaki, Y. et al., J. Pharmacol. Exp. Ther., 2002 May; 301(2):467-477
non-patent document 2: Kamada, H. et al., Nat Biotechnol., 21(4):399-404 (2003).

### [SUMMARY OF THE INVENTION]

### [Problems to be Solved by the Invention]

Under such circumstances, there is an increasing demand for the development of a carrier for drug delivery that is superior in safety and biocompatibility, and shows high kidney targeting efficiency.

The present invention aims to provide a practical carrier for drug delivery that has high biocompatibility and selectively accumulates in kidney by using a biological component, serine, as a kidney targeting element.

### [Means of Solving the Problems]

Under these circumstances, the present inventors have conducted intensive studies and found for the first time that a compound having a macromolecular carrier having a plurality of terminal groups, which is selected from the group consisting of dendrimer, dendron, dextran and chitosan, wherein the carbonyl group of serine is linked by a peptide bond or an ester bond directly or via a linker to each of at least 50% of the total number of the terminal groups (hereinafter sometimes to be referred to as "the compound of the present invention"), and a medicament containing the compound to which a drug is bonded directly or via a linker, or a drug is encapsulated therein can accomplish highly selective renal distribution and high kidney targeting efficiency, which resulted in the completion of the present invention.

Therefore, the present invention provides the following.
[1] A compound having a macromolecular carrier having a plurality of terminal groups, which is selected from the group consisting of dendrimer, dendron, dextran and chitosan, wherein the carbonyl group of serine is linked by a peptide bond or an ester bond directly or via a linker to each of at least 50% of the total number of the terminal groups.
[2] The compound of the above-mentioned [1], wherein the aforementioned macromolecular carrier is a dendrimer or a dendron.
[3] The compound of the above-mentioned [2], wherein the aforementioned dendrimer or dendron is composed of a compound selected from the group consisting of polyamidoamine, polylysine, a dendron composed of polyethylene glycol and 2,2-bis(hydroxymethyl)propanoic acid, and a dendron composed of 2,2-bis(hydroxymethyl)propanoic acid.
[4] A carrier for drug delivery that is composed of the compound of any of the above-mentioned [1] to [3], and selectively delivers a drug to a target tissue in vivo.
[5] The carrier for drug delivery of the above-mentioned [4], wherein the aforementioned target tissue is kidney.
[6] A medicament comprising the carrier for drug delivery of the above-mentioned [4] or [5], and a drug bonded to the carrier directly or via a linker or encapsulated therein.
[7] The medicament of the above-mentioned [6], wherein the aforementioned drug is at least one selected from the group consisting of an angiotensin converting enzyme inhibitor, an anti-cancer agent, an anti-inflammatory agent, an anti-infective agent, an anti-fibrotic agent, an immunosuppressant, an antioxidant, a nucleic acid drug, a radiopharmaceutical and an imaging agent.
[8] The medicament of the above-mentioned [6], wherein the aforementioned drug is captopril, and the captopril is bonded via a linker to the serine-derived terminal amino group in a serine-modified site of the aforementioned carrier for drug delivery.
[9] The medicament of the above-mentioned [6], wherein the aforementioned drug is cysteine, and the carbonyl group of the cysteine is linked by a peptide bond or an ester bond directly or via a linker to a non-serine-modified terminal group of the aforementioned carrier for drug delivery.
[10] The medicament of any of the above-mentioned [6] to [9], wherein the medicament is a prophylactic or therapeutic agent for renal diseases.

### [Effect of the Invention]

The compound of the present invention, and a medicament in which a drug is covalently or noncovalently incorporated into the compound show high renal selectivity with a particularly high renal distribution rate and scarce distribution to other organs, and are extremely useful as prophylaxis agents (test drugs) or therapeutic agents for various renal diseases. In addition, the compound of the present invention and a medicament in which a drug is bonded to or encapsulated in the compound (hereinafter sometimes to be referred to as "the medicament of the present invention" or "the pharmaceutical composition of the present invention") use biologically-derived serine as a kidney targeting element. Thus, they are superior in biocompatibility and safety, and are advantageous in that they can be synthesized easily and the like. Accordingly, they are expected to be applicable to a wide range of use as practical carriers for drug delivery that are superior in efficacy and cause less side effects.

### [Brief Description of the Drawings]

Fig. 1(a) shows the pharmacokinetics of the compound of Comparative Example 1, (b) shows the pharmacokinetics of compound 1a, (c) shows the pharmacokinetics of the compound of Comparative Example 2, and (d) shows the pharmacokinetics of the compound of Comparative Example 3.
Fig. 2 shows a weak light imaging image of organ distribution after intravenous injection of Dye800-labeled compound 1a. In Fig. 2, A shows an image of the whole body of the mouse, and B shows an image of the excised organ.
Fig. 3 shows a microscopic image of a kidney section of a mouse 60 min after intravenous administration of FITC-labeled compound 1a. In Fig. 3, (a) shows an image of renal cortex, (b) shows an enlarged image of renal cortex, and (c) shows an image of medulla.
Figs. 4A and 4B respectively show the membrane permeability of compound 1a and Comparative Example 1 in the absorption direction and secretion direction of LLC-PK1 cell monolayer membrane. Fig. 4C shows an influence of various inhibitors on the cellular uptake of ¹¹¹In-labeled compound 1a in LLC-PK1 cells.
Fig. 5 shows the pharmacokinetics of captopril after administration of captopril-compound 1a to mouse. In Fig. 5, (a) shows changes in the concentration of captopril in the plasma, (b) shows changes in the concentration of captopril in the kidney, and the captopril concentration shows the results measured by HPLC.
Fig. 6 shows changes in angiotensin converting enzyme (ACE) activity in the kidney after administration of captopril alone and administration of captopril-compound 1a.
Fig. 7 shows the pharmacokinetics of cysteine-compound 1a.
Fig. 8 shows an image of a kidney section after administration of cysteine-compound 1a in an ischemia-reperfusion mouse.
Fig. 9 shows SPECT/CT imaging images of organ distribution after intravenous injection of ¹¹¹In-labeled compound 1a.
Figs. 10A and 10B respectively show creatinine level and urea nitrogen (BUN) level in plasma of untreated, and after administration of PBS, compound 1a, or a positive control HgCl₂. C shows kidney section images of untreated, and after administration of PBS, compound 1a, or a positive control HgCl₂.

### [Description of Embodiments]

The detail of the present invention is described in the following.

### (Definition)

In the present specification, the macromolecular carrier of the "macromolecular carrier having a plurality of terminal groups" is selected from the group consisting of dendrimer, dendron, dextran and chitosan, and the terminal group means a functional group present in the terminal of the macromolecular carrier (in the case of dendrimer or dendron, it means the terminal of each branch), and may be a nucleophilic group (e.g., amino group, hydroxy group, mercapto group etc.) or electron withdrawing group (e.g., formyl group, carbonyl group etc.).

The macromolecular carrier is not particularly limited as long as it has a plurality of terminal groups. Preferred are, for example, dendrimer and dendron (e.g., 2,2-bis(hydroxymethyl)propanoic acid dendron commercially available from Sigma-Aldrich Co. LLC., etc.) each composed of polyamidoamine, polylysine, dendron composed of polyethylene glycol and 2,2-bis(hydroxymethyl)propanoic acid, or dendron composed of 2,2-bis(hydroxymethyl)propanoic acid, and one having an amino group, a hydroxy group and the like as the plurality of terminal groups. More preferred macromolecular carriers include dendrimer (PAMAM) composed of polyamidoamine having alkyldiamine (e.g., ethylenediamine, 1,4-diaminobutane, 1,6-diaminohexane, 1,12-diaminododecane etc.) as the core, dendrimer composed of polylysine having alkyldiamine (e.g., 1,6-diaminohexane etc.) as the core (e.g., polylysine dendrimer etc. described in M. Ohsaki et al., Bioconjugate Chem., 2002, 13, 510-517), and dendron composed of polylysine (e.g., polylysine dendron described in K. L. Chang et al., J. Control. Release., 2011, 156, 195-202 etc.), and one having an amino group or a hydroxy group as the plurality of terminal groups. Among those, PAMAM-NH₂ dendrimer having an amino group as the terminal group (e.g., the first generation to the fifth generation PAMAM-NH₂ dendrimers commercially available from Sigma-Aldrich Co. LLC., etc.) are particularly preferred. In addition, each constitutional unit (core and branch parts, or monomer unit of dendrimer or dendron) composing the above-mentioned macromolecular carrier is optionally substituted by substituent(s) such as C₁₋₁₂ alkyl group, halogen atom (e.g., fluorine atom etc.) and the like as long as the purpose of the present invention is not adversely affected (e.g., within the range where reaction with drug etc. does not occur).

The molecular weight of such macromolecular carrier is not particularly limited as long as it does not prevent administration to a living body. It is about 1000 - 30000 Da, preferably about 3000 - 15000 Da, more preferably about 3000 - 7000 Da.

In the present specification, "at least 50% of the total number of terminal groups" means terminal groups in the number of at least 50% (not less than 50%) of the total number of the terminal groups (that is, functional groups present at the terminals) of the aforementioned macromolecular carrier.

In the present specification, the "linker" means a bifunctional (homobifunctional or heterobifunctional) or polyfunctional chemical moiety containing a chain of atoms that covalently (by peptide bond or ester bond) connects the carboxy group of serine to the terminal group of a macromolecular carrier having a plurality of terminal groups (hereinafter sometimes to be referred to as "the first linker"), or a chain of atoms, inclusion site or chelating site that covalently or noncovalently links a drug to a serine-derived terminal amino group from among the terminal groups of the serine-modified macromolecular carrier, or the terminal group of the non-serine-modified moiety of the serine-modified macromolecular carrier (hereinafter sometimes to be referred to as "the second linker").

The bifunctional linker is preferably formed from a linker reagent having the formula:

X-R-Y

wherein X is a first reactive moiety, R is a spacer, Y is a second reactive moiety. X and Y may be suitable reactive moieties which may be the same (that is, homobifunctional linker) or different (that is, heterobifunctional linker). The suitable reactive moiety includes, but is not limited to, aldehyde (formyl group), amino group, halogen, hydroxy group, carboxy group, diene, hydrazide, maleimide, NHS ester, phosphoryl group, sulfhydryl group or other reactive moiety. Preferably, either X or Y is halogen, a formyl group, a carboxy group, hydrazide, maleimide or a sulfhydryl group. The spacer (R) may be a suitable unsubstituted or substituted aliphatic or aromatic organic moiety. The suitable organic moiety may be a divalent group selected from the group consisting of carbonyl, alkyl, cycloalkyl, cycloalkylalkyl, arylalkyl, aryl, heteroaryl, alkoxyalkyl, haloaryl, hydroxyalkyl, carboxy, carboxyalkyl, alkanoyl, alkenyl and alkynyl, but is not limited thereto. The spacer preferably contains 1 - 20 carbon atoms, more preferably 2 - 12 carbon atoms.

In the present invention, the first linker or the second linker that covalently links the carbonyl group of serine or a drug to the terminal group may be composed of one or more bifunctional linkers. As a linker reagent that forms such bifunctional linker, linker reagents commercially available from various reagent suppliers (see, for example, catalogs of reagent suppliers such as Pierce Inc., Sigma-Aldrich Co. LLC. and the like) can be mentioned. Using these reagents and according to a method known per se, the linkers can be easily introduced by those of ordinary skill in the art. Examples of the bifunctional linker include, but are not limited to, a linker, which is formed by one or more commercially available linker reagents selected from 6-maleimidocaproyl (MC), maleimidopropanoyl (MP), p-aminobenzyloxycarbonyl (PAB), ethyleneoxy(-CH₂CH₂O-; EO or PEG) as one or more repeating units, N-succinimidyl-4-(2-pyridylthio)propanoate (SPDP), pegylated long chain SPDP crosslinker, N-succinimidyl-[(N-maleimidopropionamide)-n ethyleneglycol]ester (SM(PEG)n) (wherein n is 2, 4, 6, 8, 12, 24 etc.), N-succinimidyl-4-(N-maleimidomethyl)cyclohexane-1-carboxylate (SMCC), N-succinimidyl 4-(N-maleimidomethyl)cyclohexane-1-carboxy-6-amidocaproate (LC-SMCC), 2-iminothiolane (Traut's reagent), sulfosuccinimidyl 6-(3'-(2-pyridylthio)propionamide)hexanoate (sulfo-LC-SPDP), N-succinimidyl-4-(2-pyridylthio)pentanoate (SPP), N-succinimidyl-(4-iodoacetyl)aminobenzoic acid ester (SIAB), bis-maleimido-trioxyethylene glycol (BMPEO), N-β-maleimidopropyl-oxysuccinimide ester (BMPS), N-ε-maleimidocaproyl-oxysuccinimide ester (EMCS), N-γ-maleimidobutyryl-oxysuccinimide ester (GMBS), 1,6-hexane-bis-vinylsulfone (HBVS), m-maleimidobenzoyl-N-hydroxysuccinimide ester (MBS), 4-(4-N-maleimidophenyl)butyric acid hydrazide (MPBH), succinimidyl 3-(bromoacetamide)propionate (SBAP), succinimidyl iodoacetate (SIA), succinimidyl 4-(p-maleimidophenyl)butyrate (SMPB), succinimidyl 6-((β-maleimidopropionamido)hexanoate) (SMPH), N-ε-maleimidocaproyl-oxysuccinimide ester (sulfo-EMCS), N-γ-maleimidobutyryl-oxysuccinimide ester (sulfo-GMBS), N-[K-maleimidoundecanoyloxy]-sulfosuccinimide ester (sulfo-KMUS), m-maleimidobenzoyl-N-hydroxysulfosuccinimide ester (sulfo-MBS), sulfosuccinimidyl (4-iodoacetyl)aminobenzoate (sulfo-SIAB), sulfosuccinimidyl 4-(N-maleimidomethyl)cyclohexane-1-carboxylate (sulfo-SMCC), sulfosuccinimidyl 4-(N-maleimidophenyl)butyrate (sulfo-SMPB), succinimidyl-(4-vinylsulfone)benzoate (SVSB), dithiobismaleimidoethane (DTME), 1,4-bismaleimidobutane (BMB), 1,4-bis-maleimidyl-2,3-dihydroxybutane (BMDB), bis(maleimido)hexane (BMH), bis(maleimido)ethane (BMOE), 1,8-bismaleimide-diethylene glycol (BM(PEG)₂), 1,11-bismaleimide-triethylene glycol (BM(PEG)₃), diethylenetriamine-N,N,N',N",N"-pentaacetic acid dianhydride (DTPA anhydride) and the like. Among these, a linker, which is formed by one or more linker reagents selected from the group consisting of N-succinimidyl-4-(2-pyridylthio)propanoate (SPDP), pegylated long chain SPDP crosslinker, N-succinimidyl-[(N-maleimidopropionamide)-n ethyleneglycol]ester (SM(PEG)n) (wherein n is 2, 4, 6, 8, 12, 24 etc.), N-succinimidyl 4-(N-maleimidomethyl)cyclohexane-1-carboxy-6-amidocaproate (LC-SMCC), 2-iminothiolane (Traut's reagent) and sulfosuccinimidyl 6-(3'-(2-pyridylthio)propionamide)hexanoate (sulfo-LC-SPDP) is particularly preferable.

In the present invention, examples of the second linker that retains a drug by noncovalently including in or chelating to the terminal group include a linker having a molecular recognition moiety (inclusion moiety or chelate moiety) that can be bonded to a serine-derived terminal amino group of a serine-modified macromolecular carrier, or a terminal group of a non-serine-modified moiety of a serine-modified macromolecular carrier, and can encapsulate a drug in the terminal. In such linker, an inclusion moiety or chelate moiety capable of encapsulating a drug is bonded to a serine-modified or non-serine-modified terminal group directly or via the aforementioned one or more bifunctional linkers. Such inclusion moiety or chelate moiety is not linked to a drug by a covalent bond, however, it is defined as one of the bifunctional linkers in the present specification since it can be linked to a drug by a noncovalent interaction. Examples of such inclusion moiety or chelate moiety include, but are not limited to, inclusion moieties such as 3A-amino-3A-deoxy-(2AS,3AS)-γ-cyclodextrin hydrate, cholesterol, cholic acid, C₆₀ fullerene and the like, chelate moieties such as diethylenetriamine-N,N,N',N",N"-pentaacetic acid (DTPA) and the like, and the like.

In the present specification, "the carbonyl group of serine is linked by a peptide bond or an ester bond directly or via a linker" to a terminal group of a macromolecular carrier means a state where both the respective terminal groups present in plurality in the macromolecular carrier, and a new terminal group formed by the binding of the aforementioned first linker to each terminal group are amino groups or hydroxy groups, to which the carboxy group of serine is linked by a peptide bond or an ester bond and introduced.

In the present specification, "serine-modified" and "non-serine-modified" respectively mean a state where a terminal group of a macromolecular carrier and an α-carboxy group of serine are linked by a peptide bond or an ester bond directly or via a linker, and a state where serine is not linked to a terminal group of a macromolecular carrier.

In the compound of the present invention or the medicament of the present invention, the serine-modified moiety (particularly, serine-derived hydroxy group) of a terminal group of a macromolecular carrier functions as a kidney targeting element. The "kidney targeting element" means a site having a biological recognition function and capable of forming a biological binding pair with the compound or medicament of the present invention by specifically binding to the kidney.

The introduction rate of serine (serine modification degree) into a terminal group of a macromolecular carrier that is required by the compound of the present invention or the medicament of the present invention to express high renal distribution and high renal selectivity is at least 50%, preferably not less than 60%, more preferably not less than 70%, particularly preferably not less than 80%, based on the total number of terminal groups.

In the present specification, examples of the "drug" include angiotensin converting enzyme (ACE) inhibitor, anti-cancer agent, anti-inflammatory agent, anti-infective agent, anti-fibrotic agent, immunosuppressant, antioxidant, nucleic acid drug, radiopharmaceutical, imaging agent and the like. The kind of the drug is not particularly limited. Specific examples of the drug are shown in the following, though the drug is not limited to the specific examples below.

Examples of the angiotensin converting enzyme (ACE) inhibitor include captopril, imidapril, enalapril, lisinopril, benazepril, perindopril, delapril, trandolapril, cilazapril and the like.

Examples of the anti-cancer agent include BCG, actinomycin D, asparaginase, aceglatone, anastrozole, allopurinol, anthracycline, bicalutamide, anti-androgen, idarubicin, ifosfamide, imatinib, irinotecan, interferon, interferon alpha, interleukin -2, ubenimex, exemestane, estramustine, estrogen, etoposide, enocitabine, epirubicin, oxaliplatin, octreotide, capecitabine, carboquone, carboplatin, carmofur, cladribine, clarithromycin, krestin, ketoconazole, gefitinib, gemcitabine, gemtuzumab, goserelin, cyclophosphamide, cisplatin, schizophyllan, cytarabine, cyproheptadine, zinostatin stimalamer, cetuximab, sobuzoxane, tamoxifen, daunorubicin, dacarbazine, dactinomycin, thiotepa, tegafur, tegafur-uracil, tegafur-gimeracil-oteracil potassium, dexamethasone, topotecan, trastuzumab, triptorelin, tretinoin, toremifene, doxifluridine, doxorubicin, docetaxel, nimustine, neocarzinostatin, nedaplatin, paclitaxel, hydroxyurea, hydroxycarbamide, bicalutamide, vinorelbine, vincristine, vindesine, vinblastine, picibanil, pirarubicin, fadrozole, fluorouracil, flutamide, fludarabine, busulfan, bleomycin, prednisone, procarbazine, progestin, peplomycin, pentostatin, porfimer sodium, mitomycin, mitoxantrone, mitotane, mesna, methotrexate, medroxyprogesterone, mercaptopurine, melphalan, ranimustine, rituximab, leuprolide, retinoic acid, lentinan, leucovorin and the like. The above-mentioned anti-cancer agents may be used alone, or two or more kinds of anti-cancer agents may also be used in combination.

Examples of the anti-inflammatory agent include steroidal anti-inflammatory agent and non-steroidal anti-inflammatory agent.

Examples of the steroidal anti-inflammatory agent include adrenal corticosteroidal anti-inflammatory agents, for example, dexamethasone, triamcinolone acetoide, beclometasone, hydrocortisone, methylprednisolone, predonisolone, prednisone, triamcinolone diacetate, cortisone, cortisol, paramethasone, triamcinolone, diflucortolone, difluprednate, diflorasone, flumetasone, fluocinonide, fluocinolone acetonide, alclometasone, fludrocortisone and the like, and a salt thereof. More specifically, for example, dexamethasone, triamcinolone acetonide, beclomethasone dipropionate, hydrocortisone succinate, methylprednisolone succinate, dexamethasone acetate, hydrocortisone acetate, predonisolone acetate, dexamethasone metasulfonate benzoate, triamcinolone diacetate, predonisolone butylacetate, dexamethasone phosphate, hydrocortisone phosphate, prednisolone phosphate, betamethasone phosphate, predonisolone succinate, cortisone acetate, paramethasone acetate, methylprednisolone acetate, triamcinolone, hydrocortisone, predonisolone, betamethasone, prednisolone valerate, diflucortolone valerate, dexamethasone valerate, betamethasone valerate, difluprednate acetate, diflorasone acetate, difluprednate, betamethasone dipropionate, flumetasone pivalate, fluocinonide, fluocinolone acetonide, alclometasone propionate, beclomethasone dipropionate, clobetasone butyrate, hydrocortisone butyrate, hydrocortisone butyrate propionate, fludrocortisone butyrate, dexamethasone palmitate, methylprednisolone and the like can be mentioned.

Examples of the non-steroidal anti-inflammatory agent include NSAID, COX-2 inhibitor and the like. More specifically, for example, acetylsalicylic acid, alclofenac, alminoprofen, benoxaprofen, butibufen, bucloxic acid, carprofen, celecoxib, clidanac, diclofenac, diflunisal, etodolac, fenbufen, fenoprofen, fentiazic, flufenamic acid, flufenasol, flurbiprofen, furofenac, ibufenac, ibuprofen, indomethacin, indoprofen, isoxepac, isoxicam, ketoprofen, ketorolac, meclofenamic acid, mefenamic acid, meloxicam, miroprofen, naproxen, oxaprozin, oxyphenbutazone, oxypinac, parecoxib, phenylbutazone, piclamilast, piroxicam, pirprofen, pranoprofen, rofecoxib, sudoxicam, sulindac, suprofen, tenclofenac, tiaprofenic acid, tolfenamic acid, tolmetin, tramadol, valdecoxib, zomepirac, and the like, and a salt thereof and the like can be mentioned.

Examples of the anti-infective agent include levofloxacin, ceftriaxone, minocycline, sulfamethoxazole, trimethoprim and the like.

Examples of the anti-fibrotic agent include pyridone derivative, pirfenidone, pantethine, cysteine, histidine, S-allylcysteine, growth factor, HGF (Hepatocyte growth factor), beperminogene perplasmid, osteoactivin, aldosterone antagonist, ACE inhibitor, imidapril hydrochloride, keratan sulfate oligosaccharide and the like.

Examples of the immunosuppressant include rapamycin, tacrolimus, cyclosporine, predonisolone, methylprednisolone, mycophenolate mofetil, azathioprine, mizoribine and the like.

Examples of the antioxidant include superoxide dismutase, catalase, nitric oxide donor, hydrogen sulfide donor, curcumin, coenzyme Q10, astaxanthin, α-tocopherol, α-tocopherol derivative and the like.

Examples of the nucleic acid drug include siRNA, plasmid DNA, mRNA and the like.

Examples of the radiopharmaceutical and imaging agent include yttrium Y-90, gadolinium Ga-67, gadolinium Ga-68, lutetium Lu-177, copperCu-64, technetium Tc-99, Rhenium Re-186, Rhenium Re-188, radioactive iodine-131 and the like.

In the present specification, examples of the "renal diseases" include glomerulonephritis, IgA nephropathy, diabetic nephropathy, membranous nephropathy, hydronephrosis, contrast agent nephropathy, pyelonephritis, renal failure, acute nephritis, chronic nephritis, uremia, interstitial nephritis, kidney disease, nephrotic syndrome, hypertensive nephrosclerosis, diabetic glomerulosclerosis, kidney calculus, amyloid kidney, kidney intravenous thrombosis, Alport syndrome, kidney tumor and the like.

In the present specification, moreover, the "prophylaxis or treatment" may be any prophylaxis or treatment as long as it can suppress the development of symptoms associated with renal insufficiency, inflammation of the kidney, and the like or maintain or suppress the progression thereof, and prophylaxis and treatment may not be clearly distinguished.

In the present specification, "a drug is bonded directly or via a linker" means a state where the drug is bonded by a covalent bond directly or via the aforementioned one or more bifunctional linkers (the second linker) to a serine-derived amino group on the serine-modified terminal or an amino group or a hydroxy group on a non-serine-modified terminal of the compound of the present invention.

In the present specification, "a drug is encapsulated" means a state where the aforementioned second linker having a molecular recognition moiety (e.g., inclusion moiety, chelate moiety etc.) at the terminal is bonded to a serine-derived amino group on the terminal of the compound of the present invention directly or via a linker (the aforementioned second linker), and a drug is non-covalently incorporated into the molecular recognition moiety of the compound of the present invention by hydrophobic interaction or chelating bond (i.e., included or chelated state).

The loading rate or inclusion rate of the drug in the medicament of the present invention can be appropriately changed depending on the kind of the drug. It is generally about 1 - 20%, preferably about 5 - 15%, of the total number of the terminal groups of the compound of the present invention.

The compound of the present invention is preferably the following compound.

### [Compound (A)]

The compound of the present invention having a macromolecular carrier having a plurality of terminal groups, which is selected from the group consisting of dendrimer and dendron, wherein the carbonyl group of serine is linked by a peptide bond or an ester bond directly or via a linker to each of at least 50% of the total number of the terminal groups.

### [Compound (B)]

The compound of the present invention having a macromolecular carrier having a plurality of terminal groups, which is selected from the group consisting of polyamidoamine, polylysine, a dendron composed of polyethylene glycol and 2,2-bis(hydroxymethyl)propanoic acid, and a dendron composed of 2,2-bis(hydroxymethyl)propanoic acid, wherein the carbonyl group of serine is directly linked by a peptide bond or an ester bond to each of at least 50% (preferably not less than 60%, more preferably not less than 70%, particularly preferably not less than 80%) of the total number of terminal groups.

### [Compound (C)]

The compound of the present invention having a macromolecular carrier having a plurality of terminal amino groups, which is selected from the group consisting of polyamidoamine and polylysine, wherein the carbonyl group of serine is directly linked by a peptide bond to at least 50% (preferably not less than 60%, more preferably not less than 70%, particularly preferably not less than 80%) of the total number of terminal amino groups.

The average molecular weight of the compound of the present invention is not less than 1000, preferably not less than 3000. While it does not particularly have an upper limit, not more than 40000 is desirable for easy handling.

The compound of the present invention also encompasses a salt form. Examples of the salt of the compound of the present invention include salts with inorganic acids, salts with organic acids, salts with inorganic bases, salts with organic bases, salts with amino acids and the like.

Examples of the salt with inorganic acid include salts with hydrochloric acid, nitric acid, sulfuric acid, phosphoric acid, hydrobromic acid, hydrofluoric acid, hydroiodic acid, perchloric acid and the like.

Examples of the salt with organic acid include salts with acetic acid, trifluoroacetic acid, trichloroacetic acid, propionic acid, oxalic acid, maleic acid, citric acid, fumaric acid, lactic acid, malic acid, succinic acid, tartaric acid, gluconic acid, ascorbic acid, methanesulfonic acid, trifluoromethanesulfonic acid, benzenesulfonic acid, p-toluenesulfonic acid and the like.

Examples of the salt with inorganic base include salts with sodium salt, potassium salt, calcium salt, magnesium salt, ammonium salt and the like.

Examples of the salt with inorganic base include salts with methylamine, diethylamine, trimethylamine, triethylamine, ethanolamine, diethanolamine, triethanolamine, ethylenediamine, tris(hydroxymethyl)methylamine, dicyclohexylamine, N,N'-dibenzylethylenediamine, guanidine, pyridine, picoline, choline, cinchonine, meglumine and the like.

Examples of the salt with amino acid include salts with lysine, arginine, aspartic acid, glutamic acid and the like.

The compound of the present invention also encompasses a solvate form. The solvate of the compound of the present invention also include those in the form of a solvate. The solvate of the compound of the present invention is one in which a molecule of a solvent is coordinated to the compound of the present invention, and includes a hydrate. For example, a hydrate, an ethanol solvate, a dimethyl sulfoxide solvate of the compound of the present invention or a salt thereof can be mentioned.

The compound of the present invention may be labeled with a radiopharmaceutical, an imaging agent or an isotope (e.g., ³H, ²H(D), ¹⁴C, ³⁵S, ⁹⁰Y, ¹¹¹In, ⁶⁷Ga, ⁶⁸Ga, ¹⁷⁷Lu, ⁶⁴Cu, ⁹⁹Tc, ¹⁸⁶Re, ¹⁸⁸Re, ¹³¹I, ¹⁸F etc.). As specific examples, for example, a compound obtained by introducing a chelate group (e.g., diethylenetriamine-N,N,N',N",N"-pentaacetic acid (DTPA) group etc.) into a part of the terminal group of the compound of the present invention to be ¹¹¹In chelate-labeled, a compound obtained by labeling a part of the terminal group of the compound of the present invention with fluorescein isothiocyanate (FITC), a compound obtained by labeling a part of the terminal group of the compound of the present invention with a near infrared probe (e.g., near infrared fluorescent dye, VivoTag 800 (Dye 800)) and the like are also encompassed in the compound of the present invention.

### (Synthesis of the compound of the present invention)

The production method of the compound of the present invention is not particularly limited and, for example, the compound can be synthesized by way of the following reactions.

Unless otherwise specified, the starting compounds can be easily obtained as commercially available products, or can be produced according to a method known per se (e.g., Ohsaki, M. et al., Bioconjugate Chem. 2002, 13, 510-517; Tomalia, D. A. et al., Polymer Journal, 1985, 17, 117-132; Chang, K. L. et al., J. Control. Release., 2011, 156, 195-202 etc.) or a method analogous thereto.

In each of the following steps, the protection or deprotection reaction of a functional group is performed according to a method known per se, for example, Protective Groups in Organic Synthesis, 4th Ed., Theodora W. Greene, Peter G. M. Wuts, Wiley-Interscience (2007) and the like, or a method described in the Examples of the present specification.

The compound obtained in each step in the following reaction formula can be used in the subsequent reaction as a reaction solution or as a crude product. Alternatively, the compound can also be isolated from the reaction mixture according to a conventional method, and can be easily purified by general separation means such as recrystallization, distillation, chromatography and the like.

The compound of the present invention can be produced, for example, by the following steps. 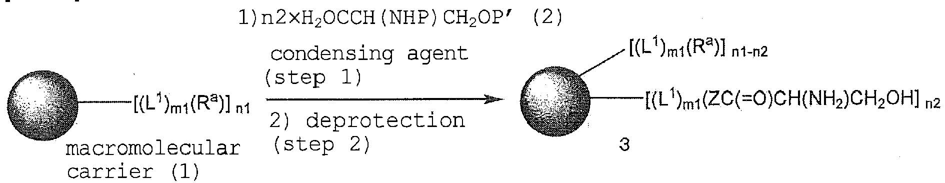 wherein L¹ is a linker (the first linker), R^{a} is an amino group or a hydroxy group, P and P' are the same or different and each independently a protecting group, Z is NH or an oxygen atom, m1 is 0 or 1, n1 is an integer of not less than 8, and n2 is an integer of not less than 4.

### Step 1

In this step, the terminal amino group or hydroxy group of macromolecular carrier (1) and an α-carboxy group of serine (compound 2) with protected amino group and protected hydroxy group are subjected to dehydration condensation directly or via the first linker at the terminal group of macromolecular carrier (1).

The reaction is carried out in a solvent that does not influence the reaction, and under dehydration condensation reaction conditions known per se.

The amount of compound 2 to be used is generally 0.5 - 3 mol, preferably 1 - 1.5 mol, based on the total number (1 mol) of terminal groups of macromolecular carrier (1).

Examples of the condensing agent to be used for the dehydration condensation reaction include 1-ethyl-3-(3'-dimethylaminopropyl)carbodiimide (WSC), dicyclohexylcarbodiimide (DCC), diisopropylcarbodiimide (DIC), N-ethyl-N'-3-dimethylaminopropylcarbodiimide and hydrochloride thereof (EDC_{•}HCl), (benzotriazol-1-yloxy)tripyrrolidino phosphonium hexafluorophosphate (PyBop), O-(benzotriazol-1-yl)-N,N,N',N'-tetramethyluronium tetrafluoroborate (TBTU), 1-[bis(dimethylamino)methylene]-5-chloro-1H-benzotriazolium 3-oxide hexafluorophosphate (HCTU), O-benzotriazole-N,N,N',N'-tetramethyluronium hexafluoroborate (HBTU) and the like.

In the condensation step, a condensation additive (e.g., 1-hydroxybenzotriazole (HOBt), 1-hydroxy-1H-1,2,3-triazole-5-carboxylic acid ethyl ester (HOCt), 1-hydroxy-7-aza benzotriazole (HOAt) etc.) and a base (e.g., organic base such as triethylamine, pyridine, N,N-diisopropylethylamine and the like, etc.) can be added where necessary.

The amount of the condensing agent to be used is generally 0.5 - 3 mol, preferably 1 - 1.5 mol, based on the total number (1 mol) of terminal groups of macromolecular carrier (1).

Examples of the solvent include aromatic hydrocarbons such as toluene, xylene and the like; amide solvents such as N,N-dimethylformamide, N,N-dimethylacetamide and the like; ethers such as diethyl ether, tetrahydrofuran, dioxane and the like; halogenated hydrocarbons such as chloroform, dichloromethane and the like and a mixture thereof. Among these, N,N-dimethylformamide, dichloromethane and the like are preferable.

The reaction temperature is generally -10 to 30°C, preferably 0°C - 20°C, and the reaction time is generally 1 to 30 hr.

### Step 2

In this step, the protecting groups P and P' of the dehydration condensation product obtained in the aforementioned step 1 are removed to synthesize compound 3.

The reaction is carried out in a solvent that does not influence the reaction, and under deprotection conditions known per se.

The reaction conditions (reaction agent, reaction solvent, reaction temperature, reaction time etc.) of deprotection vary depending on the kind of the protecting groups (P and P'). For example, it can be performed according to the method described in Protective Groups in Organic Synthesis, 4th Ed., Theodora W. Greene, Peter G. M. Wuts, Wiley-Interscience (2007), or the Examples of the present specification, or a method analogous thereto.

### (Medicament of the present invention, and a production method thereof)

A carrier for drug delivery that is composed of the compound of the present invention and selectively delivers a drug to a target tissue (kidney) in a living body is bound to the drug by a covalent bond directly or via a linker to form the medicament (compound) of the present invention, or encapsulates a drug by noncovalent interaction of a linker introduced into the carrier for drug delivery and the drug to form the medicament (composition) of the present invention.

As the medicament of the present invention, the following medicaments are preferred.

### [Medicament (A)]

The medicament of the present invention containing a drug directly bonded to a part of a serine-derived terminal amino group of a serine-modified moiety of the compound of the present invention (preferably 1 - 20%, more preferably 5 - 15%, based on the total number of serine-derived amino groups).

### [Medicament (B)]

The medicament of the present invention containing a drug directly bonded to a part of a terminal amino group or hydroxy group of a non-serine-modified macromolecular carrier moiety of the compound of the present invention (preferably 1 - 20%, more preferably 5 - 15%, based on the total number of terminal amino groups or hydroxy groups).

### [Medicament (C)]

The medicament of the present invention containing a drug bonded via one or more bifunctional linkers (the aforementioned second linkers) to a part of a serine-derived terminal amino group of a serine-modified moiety of the compound of the present invention (preferably 1 - 20%, more preferably 5 - 15%, based on the total number of serine-derived amino groups).

### [Medicament (D)]

The medicament of the present invention containing a drug bonded via one or more bifunctional linkers (the aforementioned second linkers) to a part of a terminal amino group or hydroxy group of a non-serine-modified macromolecular carrier moiety of the compound of the present invention (preferably 1 - 20%, more preferably 5 - 15%, based on the total number of terminal amino groups or hydroxy groups).

### [Medicament (E)]

The medicament of the present invention containing a drug encapsulated in a molecular recognition moiety by noncovalent interaction resulting from direct binding of a molecular recognition moiety (e.g., inclusion moiety, chelate moiety etc.) to a part of a serine-derived terminal amino group of a serine-modified moiety of the compound of the present invention (preferably 1 - 20%, more preferably 5 - 15%, based on the total number of serine-derived amino groups).

### [Medicament (F)]

The medicament of the present invention containing a drug encapsulated in a molecular recognition moiety by noncovalent interaction resulting from binding via a linker (e.g., one or more bifunctional linkers (the aforementioned second linkers)) of a molecular recognition moiety (e.g., inclusion moiety, chelate moiety etc.) to a part of a serine-derived terminal amino group of a serine-modified moiety of the compound of the present invention (preferably 1 - 20%, more preferably 5 - 15%, based on the total number of serine-derived amino groups).

When the medicament of the present invention is produced by a covalent bond formation of the compound of the present invention and a drug, as described above, it is produced by binding a drug directly or via the aforementioned one or more bifunctional linkers (the second linkers) to a serine-derived terminal amino group of a serine-modified moiety of the compound of the present invention, or directly or via the aforementioned one or more bifunctional linkers (the second linkers) to a terminal amino group or hydroxy group of a non-serine-modified macromolecular carrier moiety. The production method of such medicament is not particularly limited. In a specific embodiment, for example, as shown in the following formula: 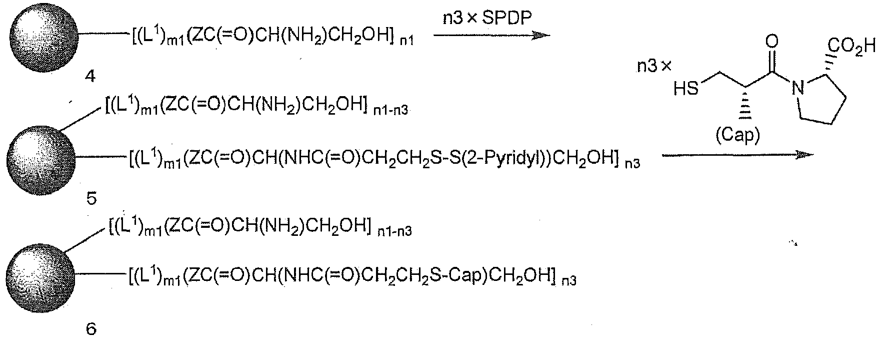 wherein Cap is captopril, SPDP is N-succinimidyl-4-(2-pyridylthio)propanoate, n3 is an integer of one or more, and other symbols are as defined above, n1>n3, a linker formed by SPDP, which is the aforementioned second linker-forming reagent, is linked according to a method known per se to a serine-derived amino group at the serine-modified terminal of the macromolecular carrier (compound 4) to give a compound having a 2-pyridyldithio group at the terminal (compound 5). Compound 5 is subjected to a disulfide bond formation reaction known per se with a drug having a sulfhydryl group at the terminal (e.g., captopril (Cap)), whereby the medicament of the present invention (compound 6) having a drug bonded to a carrier for drug delivery by an S-S bond can be produced. In addition, compound 6 can also be produced by another method below. That is, the aforementioned second linker formation reagent, SPDP, and a drug having a sulfhydryl group at the terminal (e.g., captopril (Cap)) are bonded by a disulfide bond formation reaction known per se to prepare SPDP-Cap, and then, SPDP-Cap is bonded to a serine-derived terminal amino group of a macromolecular carrier having a serine-modified terminal group (compound 4) by a method known per se, whereby the medicament of the present invention (compound 6) having the drug bonded to a carrier for drug delivery by an S-S bond can be produced.

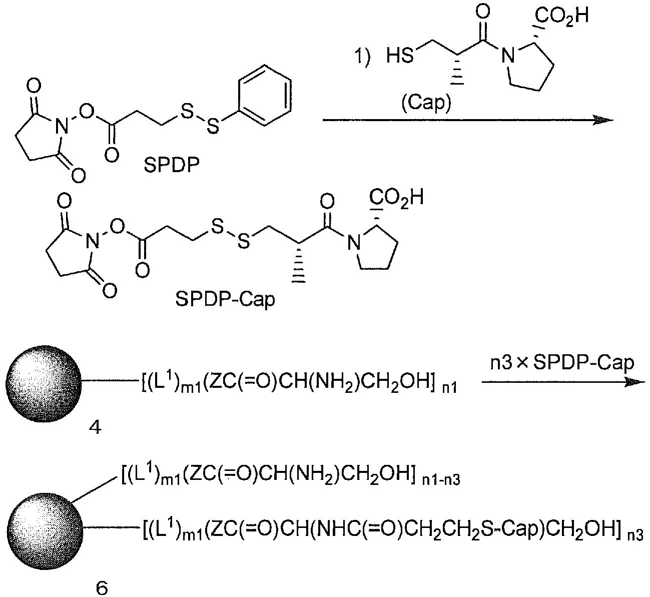

In another specific embodiment, for example, as shown in the following formula: 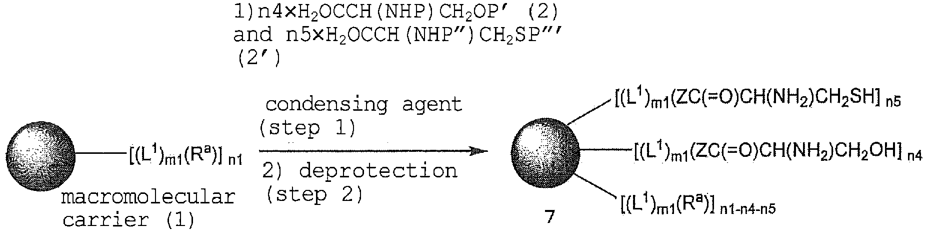 wherein P" and P"' are the same or different and each is independently a protecting group, n4 is an integer of not less than 4, n5 is an integer of one or more, and other symbols are as defined above (n1≥n4+n5), terminal amino group or hydroxy group of macromolecular carrier (1), an α-carboxy group of serine with a protected amino group and a protected hydroxy group (compound 2), and an α- carboxy group of cysteine with a protected amino group and a protected sulfhydryl group (compound 2') as a drug are subjected to dehydration condensation under the conditions of the aforementioned dehydration condensation reaction known per se, and then deprotected, whereby the medicament of the present invention (compound 7) can be produced.

When the medicament of the present invention is produced by encapsulating a drug by noncovalent interaction of a linker introduced into a carrier for drug delivery and the drug, as described above, it is produced by binding directly or via the aforementioned one or more bifunctional linkers (the second linkers) a molecular recognition moiety (e.g., inclusion moiety, chelate moiety etc.) to a serine-derived terminal amino group of a serine-modified moiety of the compound of the present invention to cause noncovalent interaction such as hydrophobic interaction, chelate binding and the like between the molecular recognition moiety and the drug, as a result of which the drug is incorporated in the carrier for drug delivery to produce the medicament of the present invention. The production method of such medicament is not particularly limited. In a specific embodiment, for example, as shown in the following formula: 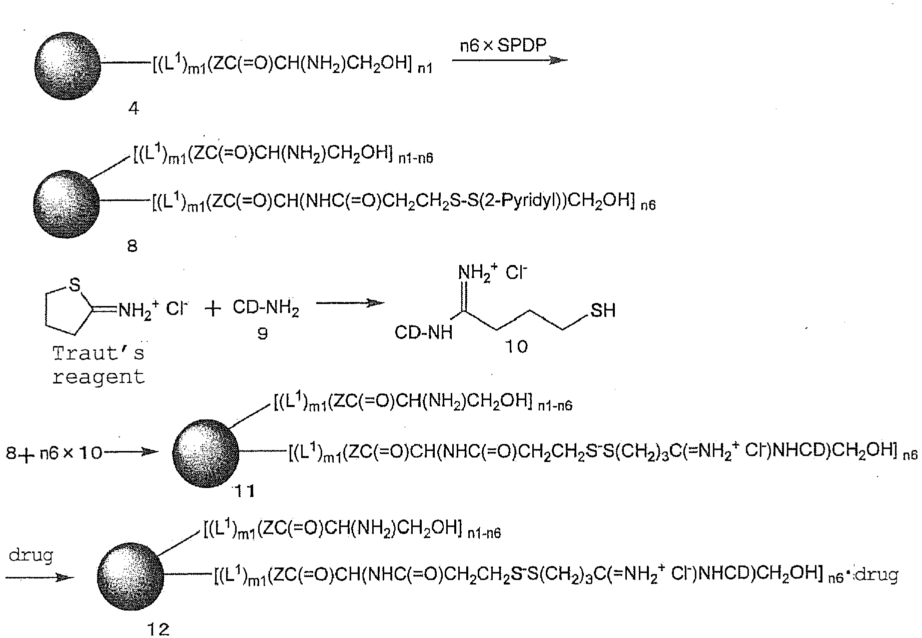 wherein CD-NH₂ is a cyclodextrin derivative having an amino group at the terminal, CD is a cyclodextrin derivative residue in which the terminal amino group is removed from CD-NH₂, n6 is an integer of one or more, and other symbols are as defined above (n1>n6), a linker formed by SPDP, which is the aforementioned second linker-forming reagent, is linked by a peptide bond to a serine-derived amino group at the terminal of the serine-modified macromolecular carrier (compound 4) to give a compound having a 2-pyridyldithio group at the terminal (compound 8). Separately, a cyclodextrin derivative having one amino side chain (compound 9) is treated with a Traut's reagent to give a compound having a sulfhydryl group at the terminal (compound 10) is obtained. Compound 8 and compound 10 are subjected to a disulfide bond formation reaction known per se to give a compound having a cyclodextrin moiety (inclusion moiety) at the terminal (compound 11), and a drug that can be included in the cyclodextrin moiety is added, whereby the medicament of the present invention (compound 12) can be produced. In another specific embodiment, for example, as shown in the following formula, though not particularly limited: 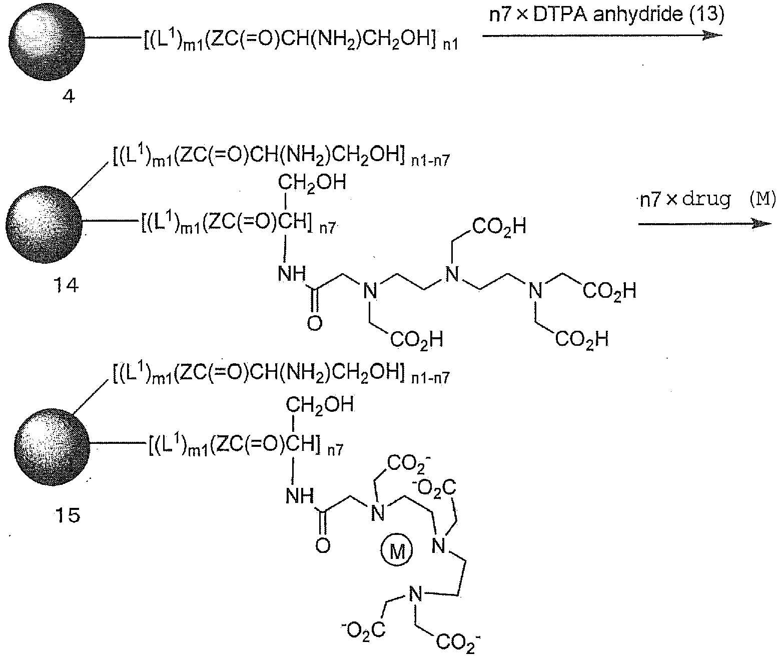 wherein M is a chelatable drug, n7 is an integer of one or more, and other symbols are as defined above (n1>n7), a linker formed by DTPA anhydride (compound 13), which is the aforementioned second linker-forming reagent, is linked by a peptide bond to a serine-derived amino group at the terminal of the serine-modified macromolecular carrier (compound 4) to synthesize compound 14, and a radioactive medicament (M) (e.g., yttrium Y-90 etc.) or the like is incorporated by chelate binding, whereby the medicament of the present invention (compound 15) can be produced. In the below-mentioned Experimental Examples 1, 4, pharmacokinetics of the compound of the present invention in which ¹¹¹In is chelated to compound 14 (¹¹¹In-labeled compound 1a) was traced.

The medicament of the present invention shows high renal distribution and can accumulate a drug selectively in the kidney. Thus, it can show superior effects as a prophylactic or therapeutic agent for various renal diseases such as renal insufficiency, inflammation of the kidney and the like. Specific examples of such renal diseases include glomerulonephritis, IgA nephropathy, diabetic nephropathy, membranous nephropathy, hydronephrosis, contrast agent nephropathy, pyelonephritis, renal failure, acute nephritis, chronic nephritis, uremia, interstitial nephritis, kidney disease, nephrotic syndrome, hypertensive nephrosclerosis, diabetic glomerulosclerosis, kidney calculus, amyloid kidney, kidney intravenous thrombosis, Alport syndrome, kidney tumor and the like.

The administration route of the medicament of the present invention is largely divided into oral administration and parenteral administration. The dose of the medicament of the present invention varies depending on the kind of the drug, administration route, administration frequency, age, body weight, pathology and severity of the subject of administration, and the like. It is generally 0.005 - 150 mg/kg/day, preferably, 0.05 - 20 mg/kg/day, and can be administered once or in several portions.

The medicament of the present invention is generally administered in the form of a pharmaceutical composition prepared by mixing with carriers for medicament. Preferable specific examples include external preparations such as microneedle, inhalant, nasal drop and the like, and injections including intracavity injections such as intravenous injection, intradermal injection, subcutaneous injection, intraperitoneal injection and the like. These pharmaceutical compositions are prepared according to conventional methods.

Specific examples the base of a microneedle include polymer bases such as polyvinylpyrrolidone, hyaluronic acid, polyglycolic acid, chondroitin sulfate, carboxymethylcellulose, maltose, dextran, polylactic acid and poly(lactic acid-co-glycolic acid), metal such as stainless steel and the like, silicon, titanium and the like. It can be produced by applying to a surface of the microneedle or placing in the inside thereof.

Inhalant can be produced by powdering or liquidifying the compound of the present invention, blending it into an inhalable spray or carrier, and filling the blend in, for example, an inhalant container such as metered-dose inhaler, dry powder inhaler and the like. It may be a propellant, an aerosol or a spray. As the inhalation propellant, those conventionally known can be widely used, and examples thereof include Freon gas such as Freon-11, Freon-12, Freon-21, Freon-22, Freon-113, Freon-114, Freon-123, Freon-142c, Freon-134a, Freon-227, Freon-C318, 1,1,1,2-tetrafluoroethane and the like, alternative Freon gas such as HFA-227, HFA-134a and the like, hydrocarbon gas such as propane, isobutane, butane and the like, diethyl ether, nitrogen gas, carbon dioxide gas and the like. As the carrier, those conventionally known can be widely used, and examples thereof include saccharides, sugar alcohols, amino acids and the like.

In the case of liquid for inhalation, it is prepared by appropriately selecting preservative (benzalkonium chloride, paraben etc.), colorant, buffering agent (sodium phosphate, sodium acetate etc.), isotonicity agent (sodium chloride, concentrated glycerol etc.), thickener (carboxyvinyl polymer etc.), preservative (benzalkonium chloride, paraben etc.), absorption promoter and the like as necessary.

In the case of powder for inhalation, it is prepared by appropriately selecting lubricant (stearic acid and a salt thereof etc.), binder (starch, dextrin etc.), excipient (lactose, cellulose etc.), colorant, absorption promoter and the like as necessary.

Nasal drop can take various forms such as a dropping type, an application type, a spray type and the like. In the case of the spray type, a manual pump type nasal drop with a mechanism to eject the liquid by manually moving the pump attached to the container, an aerosol-type nasal drop having a mechanism to automatically eject a liquid agent by filling the container with a propellant agent and moving a valve attached to the container and the like are also included.

Injection is prepared by dissolving the medicament of the present invention in distilled water for injection, and further, a solution containing a solubilizing agent, a buffer, a pH adjuster, an isotonic agent, a soothing agent, a preservative, and the like, where necessary, or suspending the compound in distilled water for injection or vegetable oil. In this case, a base, a suspending agent, a thickener and the like can be added as necessary. In addition, it may be in a form for dissolving a powder or a freeze-dried product when in use, and an excipient (e.g., mannitol, sorbitol, lactic acid, trehalose, sucrose etc.) and the like can be added as necessary.

The content of the medicament of the present invention in a pharmaceutical composition varies depending on the dosage form thereof. It is generally 0.0025 - 30 wt% of the whole composition. Such pharmaceutical composition may also contain other drugs effective for treatment. In combined use, the administration period of the compound of the present invention and a concomitant drug is not particularly limited, and the mixing ratio of the medicament of the present invention and the concomitant drug can be appropriately determined according to the subject of administration, administration method, disease, combination and the like. For example, while the content of a concomitant drug in the combination drug of the present invention varies depending on the form of the preparation, it is generally 0.0025 - 30 wt% of the whole composition.

While the present invention is described in more detail in the following preferred Examples and Experimental Examples of the present invention, the following Examples are only for exemplary confirmation of the effects of the present invention and do not limit the present invention to them. In addition, the present invention may be modified without departing from the scope of the invention.

### [Examples]

The present invention is explained in more detail in the following by referring to Examples, Formulation Example and Experimental Examples, which do not limit the present invention. In addition, the present invention may be modified without departing from the scope of the invention.

Unless otherwise specified, the apparatus, reagents, and the like used in the Examples can be easily obtained or prepared according to a method generally employed in the art, or are commercially available.

The "room temperature" in the following Examples is generally about 10°C to about 25°C.

### Example 1: Synthesis of kidney targeting carrier for drug delivery (serine-modified dendrimer) (compound of the present invention (compound 1a))

1.1 equivalents of Boc-Ser(tBt)-OH (manufactured by Watanabe Chemical Industries, Ltd.), 1.1 equivalents of 1-[bis(dimethylamino)methylene]-1H-benzotriazolium 3oxide hexafluorophosphate (HBTU) (manufactured by Merck Millipore), 1.1 equivalents of anhydrous 1-hydroxy-1H-benzotriazole (HOBt) (manufactured by Watanabe Chemical Industries, Ltd.) and 2.2 equivalents of N,N-diisopropylethylamine (DIPEA), each to the total number of surface amino groups of the third generation polyamidoamine dendrimer (PAMAM) (manufactured by Sigma-Aldrich), were mixed in DMF/DMSO (1:1). Then, the reaction mixture was reacted by stirring at room temperature until the ninhydrin test yielded negative results on TLC analysis. After completion of coupling, this solution was purified by precipitation with diethylether three times. The precipitates thereof were dissolved in a trifluoroacetic acid (TFA) cocktail (95% TFA, 2.5% thioanisole (TIS) and 2.5% purified water) to deprotect the Boc and OBt groups. Then, the reaction mixture was incubated at room temperature for 90 min. After completion of deprotection, the reaction mixture was purified by precipitation with diethylether three times. The crude precipitates were dissolved in ultrapure water and passed through PD-10 column to separate the resulting products by size-exclusion chromatography, and then lyophilized to obtain serine-modified PAMAM (hereinafter to be referred to as "compound 1a"). For the analysis of particles size and zeta potential, compound 1a was dissolved in phosphate buffered saline (PBS) (pH 7.4) at a concentration of 1 mg/mL. Then, these properties were measured by Zetasizer Nano ZS (Malvern Instruments, Worcestershire, UK) for the particle size and zeta potential of compound 1a.

The particle size of the obtained compound 1a was about 4 nm (4.03±0.09 nm), and the zeta potential was about 4.8 mV (4.76±0.70 mV).

### Comparative Example 1: polyamidoamine dendrimer (PAMAM G4)

The fourth generation polyamidoamine dendrimer (PAMAM G4) (manufactured by Sigma-Aldrich) was used as the compound of Comparative Example 1.

The particle size of the compound of Comparative Example 1 was about 4.2 nm (4.20±0.09 nm), and the zeta potential was about 4.6 mV (4.56±0.81 mV).

### Comparative Example 2: Synthesis of threonine-modified dendrimer

By a method similar to Example 1 except that Boc-Ser(tBt)-OH used in Example 1 was changed to Boc-Thr(tBu)-OH (manufactured by Watanabe Chemical Industries, Ltd.), the compound of Comparative Example 2 was prepared.

The particle size of the compound of Comparative Example 2 was about 4.2 nm (4.15±0.35 nm), and the zeta potential was about 2.6 mV (2.58±1.36 mV).

### Comparative Example 3: Synthesis of tyrosine-modified dendrimer

By a method similar to Example 1 except that Boc-Ser(tBt)-OH used in Example 1 was changed to Boc-Tyr(tBu)-OH (manufactured by Watanabe Chemical Industries, Ltd.), the compound of Comparative Example 3 was prepared.

The particle size of the compound of Comparative Example 3 was about 3.2 nm (3.17±0.35 nm), and the zeta potential was about 5.3 mV (5.26±3.00 mV).

### Example 2: Synthesis of captopril-bonded compound 1a (captopril-compound 1a)

6 equivalents of N-succinimidyl-4-(2-pyridylthio)propanoate (SPDP) (manufactured by Tokyo Chemical Industry Co., Ltd.) and 6.6 equivalents of captopril (manufactured by Tokyo Chemical Industry Co., Ltd.), each to compound 1a, were added to DMSO, and the mixture was stirred at room temperature for 10 min to allow for reaction of SPDP and captopril. Thereafter, the reaction mixture was mixed with compound 1a dissolved in DMSO and the mixture was stirred at room temperature for 12 hr, captopril was bonded via SPDP to the amino group at the terminal of compound 1a to synthesize captopril-bonded compound 1a (captopril-compound 1a).

### Example 3: Synthesis of cysteine-bonded compound 1a (cysteine-compound 1a)

0.88 equivalents of Boc-Ser (tBt)-OH, 0.22 equivalents of Boc-Cys(Trt)-OH (manufactured by Watanabe Chemical Industries, Ltd.), 1.1 equivalents of 1-[bis(dimethylamino)methylene]-1H-benzotriazolium 3oxide hexafluorophosphate (HBTU), 1.1 equivalents of anhydrous 1-hydroxy-1H-benzotriazole (HOBt) and 2.2 equivalents of N,N-diisopropylethylamine (DIPEA), each to the total number of surface amino groups of the third generation polyamidoamine dendrimer (PAMAM), were mixed in DMF/DMSO (1:1). The synthetic step thereafter was performed by a method similar to that in Example 1.

### Experimental Example 1: Evaluation of pharmacokinetics of compound 1a

### (Test method)

¹¹¹In-labeled compound 1a (hereinafter to be also referred to as "¹¹¹In-labeled compound 1a") was intravenously administered to ddY mice, and pharmacokinetics of compound 1a were evaluated. To be specific, compound 1a was radiolabeled with ¹¹¹In by using a bifunctional chelating agent, diethylenetriamine-N,N,N',N",N"-pentaacetic acid (DTPA) anhydride (manufactured by DOJINDO LABORATORIES), and according to the method described in Hnatowich et al. (Int. J. Appl. Radiat. Isot., 12, 327-332 (1982)). The obtained ¹¹¹In-labeled compound 1a was intravenously administered to ddY mice via the tail vein. At a suitable time point after intravenous injection, the blood was collected from the abdominal vena cava under isoflurane anesthesia. Liver, kidney, spleen, heart and lung tissue were excised, rinsed with saline, blotted dry, and the wet weight of the organs was measured. The collected blood was centrifuged at 2000×g for 5 min to obtain plasma. The samples of the collected organs and 100 µL of plasma were transferred to counter tubes, and the radioactivity of each sample was measured using a gamma counter (1480 WizardTM 3', Perkin-Elmer, Boston, MA, USA).

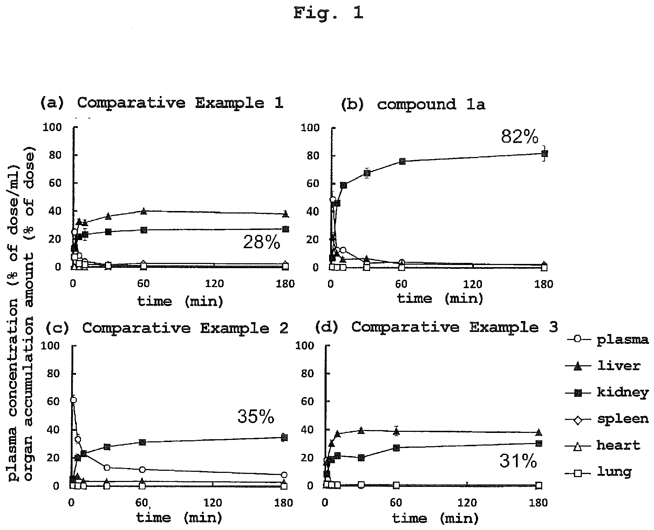

Fig. 1 shows the concentration-time profiles of plasma, liver, kidney, spleen, heart and lung after intravenous injection of ¹¹¹In-labeled compound 1a (i.e., pharmacokinetics of compound 1a).

According to Fig. 1(b), about 80% of the dose of compound 1a after intravenous administration was accumulated in kidney. Accumulation in other organs was scarcely found, and the compound showed pharmacokinetics superior in renal selectivity. In contrast, non-serine-modified PAMAM (compound of Comparative Example 1) (Fig. 1(a)) and the compound of Comparative Example 2 (Fig. 1(c)) and the compound of Comparative Example 3 (Fig. 1(d)), each of which was modified with threonine (Thr) or tyrosine (Tyr) having structures similar to that of serine, did not show renal distribution or renal selectivity equivalent to that of compound 1a. To be specific, the compound of Comparative Example 1 was mainly distributed in liver and kidney and showed a low renal distribution rate, the compound of Comparative Example 2 was mainly distributed in kidney and showed a low renal distribution rate, and the compound of Comparative Example 3 was mainly distributed in liver and kidney and showed a low renal distribution rate.

From the results of Fig. 1, it was confirmed that compound 1a is particularly superior in renal distribution and renal selectivity.

### Experimental Example 2: Organ distribution of compound 1a by weak light imaging system

### (Test method)

The compound of Comparative Example 1 and compound 1a were labeled with a near infrared fluorescence dye, Vivo Tag 800 (Dye800) (manufactured by PerkinElmer Co., Ltd.). That is, compound 1a was dissolved in 50 mM carbonate/bicarbonate buffer (pH 8.5), and Dye800 in DMSO was added to compound 1a at a molar ratio of 2:3. After mixing at room temperature for 1 hr, the reaction mixture was purified by ultra-filtration. Then, a solution of Dye800-labeled compound 1a (Dye800-labeled compound 1a) was intravenously administered to HR-1 mouse at a dose of 1 mg/kg. At 60 min after intravenous administration, mouse systemic imaging was performed using IVIS Lumina XRMS Series III Multi-Species Optical and X-Ray Imaging System under isoflurane anesthesia. Thereafter, under isoflurane anesthesia, the blood was collected from the abdominal vena cava. The mouse was perfused with 10 ml of saline via left ventricle of the heart to wash out compound 1a in the blood, and the liver, kidney, spleen, heart and lung tissue were excised together with the bone of the lower leg and washed with saline. Successively, imaging was performed using IVIS Lumina XRMS Series III Multi-Species Optical and X-Ray Imaging System.

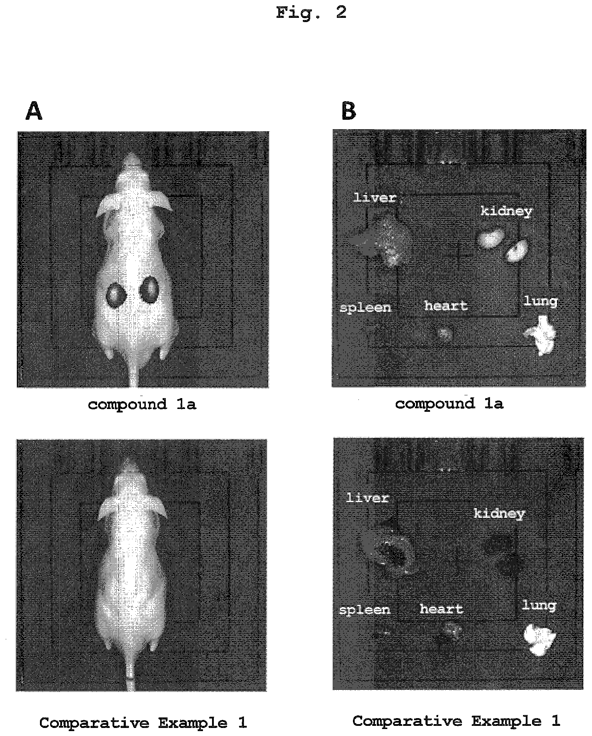

Fig. 2 shows a weak light imaging image of organ distribution after intravenous injection of Dye800-labeled compound 1a. Fig. 2A shows an image of the whole body of the mouse, and Fig. 2B shows an image of the excised organ. According to Fig. 2, Dye800-labeled compound 1a after intravenous administration was mainly accumulated in kidney and efficient kidney targeting was confirmed.

### Experimental Example 3: Distribution of compound 1a in kidney

### (Test method)

Compound 1a was labeled with fluorescein isothiocyanate (FITC) (manufactured by Sigma-Aldrich) (FITC-labeled compound 1a). FITC-labeled compound 1a was administered to ddY mice from the tail vein. At 60 min after intravenous administration, the kidneys were excised under isoflurane anesthesia, fixed with OCT, and frozen. Frozen kidney sections were treated with 100 µg/mL 4',6-diamidino-2-phenylindole (DAPI) (manufactured by Wako Pure Chemical Industries, Ltd.), and stained kidney sections were observed under a fluorescence microscope (Biozero, KEYENCE, Osaka, Japan).

### (Results)

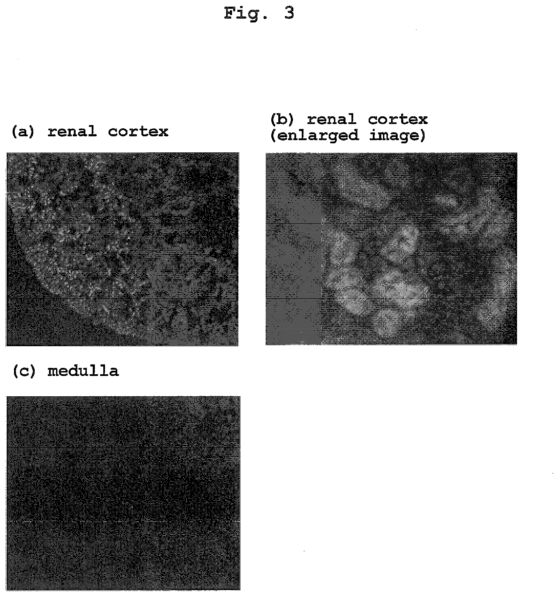

Fig. 3 shows a microscopic image of a kidney section of a mouse 60 min after intravenous administration of FITC-labeled compound 1a. According to Fig. 3(a), it was confirmed that the fluorescence of FITC-labeled compound 1a was remarkably high in the renal cortex of renal medulla. An enlarged microscopic image of renal cortex is shown in Fig. 3(b). Strong fluorescence derived from FITC-labeled compound 1a was observed in the proximal tubules. On the other hand, fluorescence derived from FITC-labeled compound 1a was scarcely confirmed in medulla. From the above, it was confirmed that compound 1a is mainly distributed in the proximal tubules of kidney. Since proximal tubule is the site of onset of renal diseases such as kidney cancer, chronic renal failure and the like, compound 1a was found to show distribution in kidney which is advantageous for the treatment of renal diseases and image diagnosis.

### Experimental Example 4: Analysis of membrane permeability and renal uptake mechanism of compound 1a by using swine kidney epithelial cell

### (Test method)

To evaluate kidney epithelial cell membrane permeability (permeability in absorption direction or secretion direction) of compound 1a, the permeability of compound 1a through a single layer membrane of LLC-PK1 cells (swine kidney epithelial cells) was evaluated. That is, LLC-PK1 cells were seeded on Transwells (Corning Inc, Corning, NY) at 2.5×10⁵ cells/insert, and cultured for 1 week. The apical and basolateral sides were pre-incubated with Hank's balanced salt solution (HBSS; pH 7.4) for 1 hr. Then, 0.1 mg/mL of FITC-labeled compound 1a in HBSS was added to the apical side (0.5 mL) or basolateral side (1.5 mL). 100 µL of receptor solution (apical side when FITC-labeled compound 1a was added to basolateral side, and basolateral side when FITC-labeled compound 1a was added to apical side) was recovered over time, an equal amount of HBSS was added to the receptor side. The amount of FITC-compound 1a was measured using a fluorescence spectrophotometer (PowerScan HT, BioTek Instruments). Excitation wavelength used was 485 nm, and the fluorescence wavelength used was 528 nm.

Separately, cell uptake mechanism of compound 1a in kidney epithelial cells was evaluated. That is, LLC-PK1 cells were seeded on a collagen-coated 24 well plate at 2.5×10⁵ cells/insert, and cultured for 1 week. The cells were pre-incubated in HBSS (pH 7.4) for 1 hr, then, 5 µg/mL of ¹¹¹In-labeled compound 1a was added, and the intracellular uptake amount of compound 1a was evaluated with intracellular radioactivity as an index. Various inhibitors (excess amount of unlabeled compound 1a, chlorpromazine 100 µM, genistein 370 µM, 5-(N-ethyl-N-isopropyl)amiloride 100 µM and lysozyme 1 mM) were simultaneously added, and the uptake mechanism was evaluated by a decrease in the uptake amount of compound 1a.

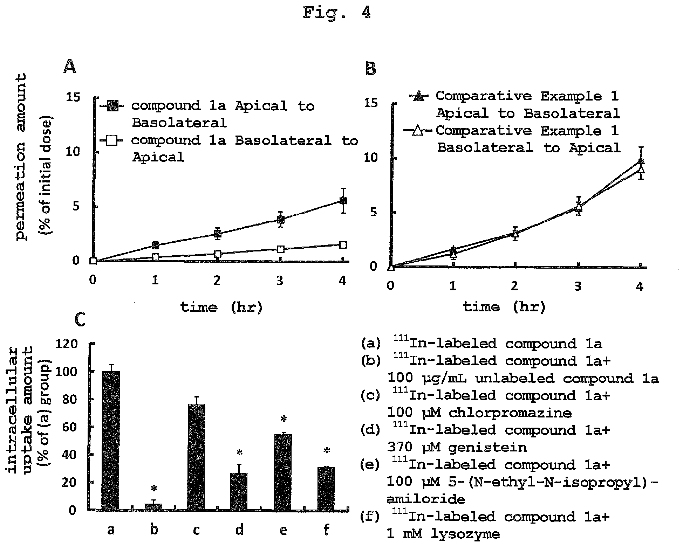

The results are shown in Fig. 4.

Fig. 4A and Fig. 4B respectively show the membrane permeability of compound 1a and Comparative Example 1 in the absorption direction and secretion direction of LLC-PK1 cell monolayer membrane. The membrane permeability in Comparative Example 1 was equivalent in the absorption direction and the secretion direction, and no difference was found. In contrast, the membrane permeability of compound 1a was higher in the absorption direction than in the secretion direction, and permeability superior in the absorption direction was found. Therefrom it is considered that compound 1a is incorporated from the renal tubule luminal side to the blood vessel side.

Fig. 4C shows influence of various inhibitors on the intracellular uptake of ¹¹¹In-labeled compound 1a in LLC-PK1 cells. A significant decrease in the intracellular uptake of compound 1a was found in a group added with an excess amount of unlabeled compound 1a, a group added with a caveolae-dependent endocytosis inhibitor, genistein, a group added with a macropinocytosis inhibitor, 5-(N-ethyl-N-isopropyl)amiloride, and a group added with megalin substrate lysozyme. Thus, it was suggested that compound 1a may be incorporated by the contribution of caveolae-dependent endocytosis, macropinocytosis or megalin-mediated transcytosis.

### Experimental Example 5: Pharmacokinetics of captopril-compound 1a

### (Test method)

The pharmacokinetics of captopril-compound 1a was evaluated by intravenous administration of captopril alone or captopril-compound 1a to ddY mice each at a dose of 2 mg captopril/kg. At a suitable time point after intravenous administration, the blood was collected from the abdominal vena cava under isoflurane anesthesia and the kidney was excised. Thereafter, the captopril concentrations in the plasma and kidney homogenate were measured using HPLC.

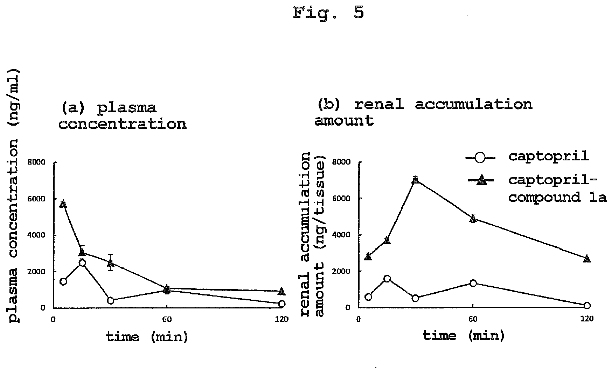

The results are shown in Fig. 5. According to Fig. 5(b), the concentration of captopril in the kidney after administration of captopril-compound 1a is remarkably high compared to that of captopril alone administration group. Therefrom it was confirmed that captopril is efficiently accumulated in the kidney by utilizing compound 1a.

### Experimental Example 6: Pharmacological effect of captopril-compound 1a

### (Test method)

Captopril or captopril-compound 1a was intravenously administered to ddY male mice each at a dose of 0.5 mg captopril/kg. At 30 min and 120 min after administration, the kidney was excised under isoflurane anesthesia, homogenized in 2 mL of 50 mM KHPO₄ buffer (pH 7.5), and the homogenate was centrifuged. The supernatant solution (100 µL), ultrapure water (100 µL) and 50 mM N-hippuryl-L-histidyl-L-leucine (50 µL) were mixed and reacted at 37°C for 15 min, and the enzyme reaction was discontinued with 1N HCl (250 µL). Hippuric acid was extracted with ethyl acetate (400 µL×2 times), and ethyl acetate was transpirated. The level of the obtained hippuric acid was measured by HPLC, and angiotensin-converting enzyme (ACE) activity was evaluated with the hippuric acid level as an index.

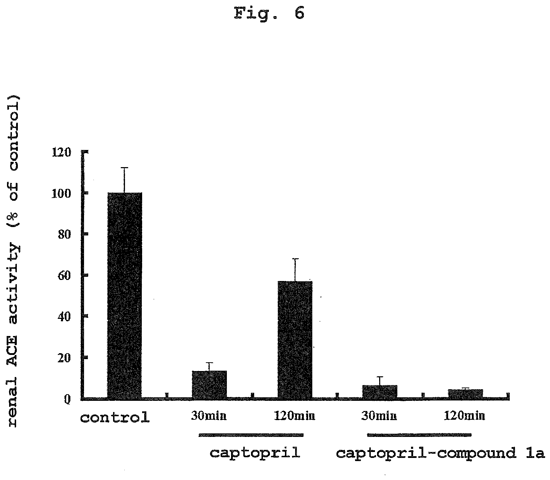

The results are shown in Fig. 6. According to Fig. 6, it was found that the activity of ACE in the kidney is suppressed in a sustained manner by the administration of captopril-compound 1a compared to the administration of captopril alone.

### Experimental Example 7: Evaluation of pharmacokinetics of cysteine-compound 1a

### (Test method)

¹¹¹In-labeled cysteine-compound 1a was intravenously administered to ddY mice, and the pharmacokinetics of cysteine-compound 1a was evaluated. The cysteine-compound 1a was labeled with ¹¹¹In by a method similar to that in Experimental Example 1.

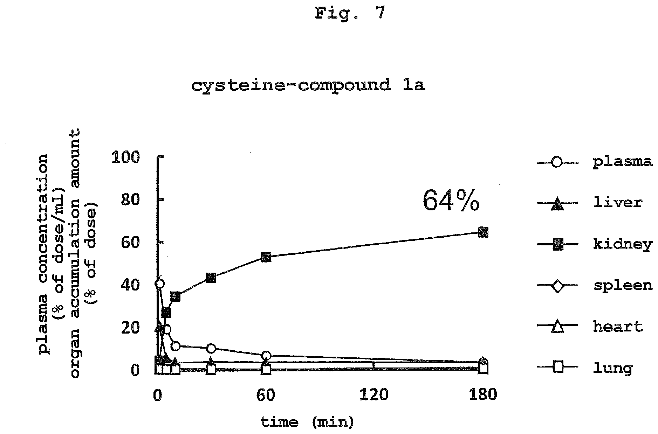

The results are shown in Fig. 7. According to Fig. 7, cysteine-compound 1a, like compound 1a, was not accumulated in other organs, and showed kidney-selective pharmacokinetics.

### Experimental Example 8: Kidney protective effect of cysteine-compound 1a against kidney ischemia reperfusion disorder

### (Test method)

Under isoflurane anesthesia, the right kidney was excised from ddY mice, the blood flow of renal artery of the left kidney was blocked with klemme, the blood flow was resumed 30 min later to create a renal ischemia-reperfusion injury model. Cysteine-compound 1a was intravenously administered immediately before resumption of the blood flow, and the degree of renal damage was evaluated from a kidney section image at 6 hr after resumption of the blood flow.

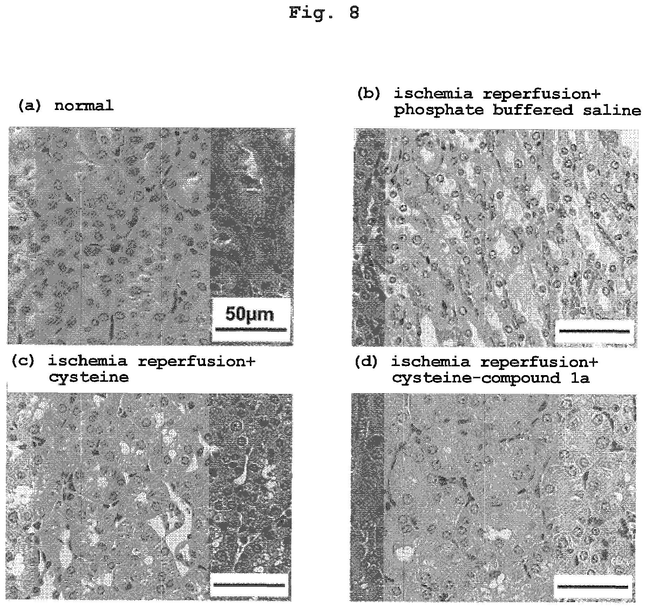

The results are shown in Fig. 8. According to Fig. 8, morphological changes of kidney were observed by renal ischemia-reperfusion. However, it was confirmed that the administration of cysteine-compound 1a remarkably suppressed the morphological changes.

### Experimental Example 9: Observation of organ distribution of compound 1a by single photon emission computed tomography/computed tomography (SPECT/CT) imaging

### (Test method)

SPECT/CT was performed using NanoSPECT/CT (Bioscan Inc., Washington DC, USA). ¹¹¹In-labeled compound 1a (8.7 MBq/mouse) was injected intravenously into the mouse. At 3 hr after intravenous administration of ¹¹¹In-labeled compound 1a, under isoflurane anesthesia, a 45 min SPECT scan of the mouse was obtained. Prior to the SPECT scan, under isoflurane inhalation anesthesia, a CT scan of the mouse was performed for anatomical reference. The SPECT image was reconstructed using HiSPECT software (Scivis, Goettingen, Germany). Also, using the Amira 3D data analysis and visualization software package (version 5.1; FEI Company, Hillsboro, OR, USA), image analysis was performed.

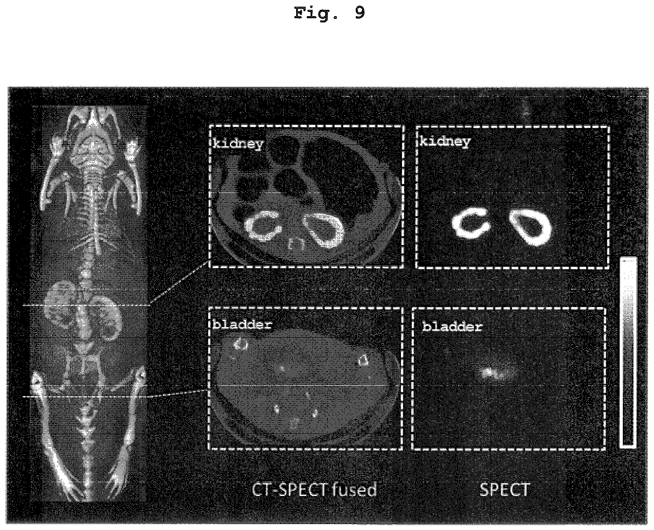

The results are shown in Fig. 9. Fig. 9 shows SPECT/CT imaging images of organ distribution after intravenous injection of ¹¹¹In-labeled compound 1a. According to Fig. 9, ¹¹¹In-labeled compound 1a was partly distributed to the bladder (excreted in urine) but specifically accumulated in the kidney. In the kidney, ¹¹¹In-labeled compound 1a was mainly accumulated in the cortex part where proximal tubules and the like are present. Since proximal tubule is the site of onset of renal diseases such as renal cell carcinoma, chronic renal failure and the like, these study results suggest that compound 1a may be applicable to image diagnosis of renal diseases.

### Experimental Example 10: Evaluation of toxicity of compound 1a in mouse

### (Test method)

Compound 1a (1 mg/kg) or PBS was intravenously administered to ddY mice once per day for 5 days. Six days after the first intravenous injection, under isoflurane anesthesia, the blood was collected from the vena cava to sacrifice the mice. Plasma creatinine and urea nitrogen (BUN) levels were respectively measured using a creatinine measurement kit (LabAssay, Wako) and a BUN measurement kit (DIUR-100, BioAssay System). Thereafter, kidneys were excised, fixed with 4% paraformaldehyde, and embedded in paraffin blocks. Then, 5-µm-thick sections were prepared. The kidney sections were stained with hematoxylin and eosin, and renal injury was evaluated using a light microscopy (Biozero, KEYENCE). In addition, when HgCl₂ was administered as a positive control, plasma creatinine, BUN level and kidney sections were also evaluated by the above-mentioned methods.

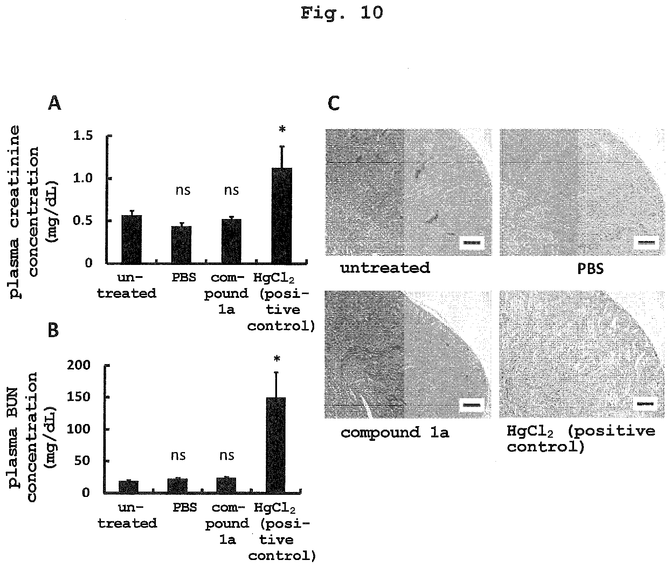

The results are shown in Fig. 10. Plasma concentrations of creatinine and BUN significantly increased by subcutaneous injection of HgCl₂ as a positive control (Figs. 10A and 10B). Furthermore, infiltration and necrosis of inflammatory cells and damaged cells were observed in the histological sections of the kidney tissues derived from ddY mice administered with HgCl₂ (Fig. 10C). In contrast, administration of compound 1a scarcely influenced plasma concentrations of creatinine and BUN. The histological sections of renal tissues after administration of compound 1a were similar to those of untreated and PBS-administered mice (Fig. 10C). The foregoing results reveal that compound 1a is a drug carrier with relatively high safety.

**Formulation Example (production of freeze-dried preparation)**

| | |
|---|---|
| 1) Medicament of the present invention (captopril-compound 1a) | 40 mg |
| 2) Mannitol | 10 mg |
| 3) Ultrapure water | 1 ml |
| Total | 50 mg/ml |

1), 2) and 3) were mixed, sterilized by filtration with a membrane filter, filled in a container and lyophilized to give a freeze-dried preparation.

### [Industrial Applicability]

The compound of the present invention, and a medicament in which a drug is covalently or noncovalently incorporated into the compound show high renal selectivity with a particularly high renal distribution rate as compared to prior art and scarce distribution to other organs, and are extremely useful as prophylaxis agents (test drugs) or therapeutic agents for various renal diseases. In addition, the compound and the medicament of the present invention use biologically-derived serine as a kidney targeting element. Thus, they are superior in biocompatibility and safety and can be synthesized easily. Accordingly, they are expected to be applicable to a wide range of use as practical carriers for drug delivery that are superior in efficacy and cause fewer side effects.

This application is based on a patent application No. 2017-132806 filed in Japan, the contents of which are incorporated in full herein.

## Claims

1. A compound having a macromolecular carrier having a plurality of terminal groups, which is selected from the group consisting of dendrimer, dendron, dextran and chitosan, wherein the carbonyl group of serine is linked by a peptide bond or an ester bond directly or via a linker to each of at least 50% of the total number of the terminal groups.

2. The compound according to claim 1, wherein the aforementioned macromolecular carrier is a dendrimer or a dendron.

3. The compound according to claim 2, wherein the aforementioned dendrimer or dendron is composed of a compound selected from the group consisting of polyamidoamine, polylysine, a dendron composed of polyethylene glycol and 2,2-bis(hydroxymethyl)propanoic acid, and a dendron composed of 2,2-bis(hydroxymethyl)propanoic acid.

4. A carrier for drug delivery that is composed of the compound according to any one of claims 1 to 3, and selectively delivers a drug to a target tissue in vivo.

5. The carrier for drug delivery according to claim 4, wherein the aforementioned target tissue is kidney.

6. A medicament comprising the carrier for drug delivery according to claim 4 or 5, and a drug bonded to the carrier directly or via a linker or encapsulated therein.

7. The medicament according to claim 6, wherein the aforementioned drug is at least one selected from the group consisting of an angiotensin converting enzyme inhibitor, an anti-cancer agent, an anti-inflammatory agent, an anti-infective agent, an anti-fibrotic agent, an immunosuppressant, an antioxidant, a nucleic acid drug, a radiopharmaceutical and an imaging agent.

8. The medicament according to claim 6, wherein the aforementioned drug is captopril, and the captopril is bonded via a linker to the serine-derived terminal amino group in a serine-modified site of the aforementioned carrier for drug delivery.

9. The medicament according to claim 6, wherein the aforementioned drug is cysteine, and the carbonyl group of the cysteine is linked by a peptide bond or an ester bond directly or via a linker to a non-serine-modified terminal group of the aforementioned carrier for drug delivery.

10. The medicament according to any one of claims 6 to 9, wherein the medicament is a prophylactic or therapeutic agent for renal diseases.
